# EUROPEAN PATENT SPECIFICATION

(11) **EP 1 698 631 B1**
(45) Date of publication and mention of the grant of the patent: **10.08.2011**
(21) Application number: 04807680.6
(22) Date of filing: 24.12.2004
(51) Int. Cl.: C07F 5/02, C07C 211/63, C07D 295/02, C07D 295/08, H01G 9/038, H01M 6/16

(54) **IONIC LIQUID, METHOD FOR PRODUCING SAME, DOUBLE LAYER CAPACITOR COMPRISING SAME, AND LITHIUM BATTERY**
IONISCHE FLÜSSIGKEIT, VERFAHREN ZU DEREN HERSTELLUNG, DOPPELSCHICHTIGER KONDENSATOR, DER DIESE ENTHÄLT, UND LITHIUMBATTERIE
LIQUIDE IONIQUE, SON PROCEDE DE PREPARATION, CONDENSATEUR DOUBLE COUCHE COMPRENANT CE LIQUIDE ET PILE AU LITHIUM

(30) Priority: 26.12.2003 JP 2003431700; 27.01.2004 JP 2004019074; 27.01.2004 JP 2004019076; 29.03.2004 JP 2004094275; 29.03.2004 JP 2004094293; 30.09.2004 JP 2004285706
(43) Date of publication of application: 06.09.2006
(73) Proprietor: National Institute of Advanced Industrial Science and Technology, Tokyo 100-8921 (JP)
(72) Inventor: MATSUMOTO, H., 8-31, Midorigaoka 1-chome,Ikeda-shi, Osaka 5638577 (JP); ZHOU, Z., 8-31 Midorigaoka 1-chome,Ikeda-shi, Osaka 5638577 (JP)
(74) Representative: Smyth, Gyles Darren
(86) International application number: PCT/JP2004/019323
(87) International publication number: WO 2005/063773

(56) References cited:
- EP-A2- 1 205 480
- WO-A-02/076924
- JP-A- 2002 100 403
- JP-A- 2004 175 667
- ZHOU Z-B. ET AL: 'A new class of hydrophobic ionic liquids: Trialkyl-(2-methoxyethyl) ammonium perfluoroethyltrifluoroborate' CHEMISTRY LETTERS vol. 33, no. 7, 2004, pages 886 - 887, XP002986344

## Description

### TECHNICAL FIELD

The present invention relates to ionic liquids, and more particularly to ionic liquids with low viscosities and melting points as well as high conductivities and electrochemical stabilities. The present invention also relates to a method of producing ionic liquids as well as lithium batteries (for example, lithium-ion batteries, lithium primary batteries and lithium secondary batteries, and particularly lithium secondary batteries) and electric double-layer capacitors comprising the ionic liquids.

### PRIOR ART

Ionic liquids have attracted special attention for the past several years, owing to their potential for application as the electrolytes, reaction media and catalysts for organic syntheses for a variety of electrochemical devices, such as lithium secondary batteries, solar cells, actuators, electric double-layer capacitors and the like. Compared with conventional organic liquid electrolytes, ionic liquids as such electrolytes have the main advantages of flame retardancy, non-volatility and high thermal stability. Bistrifluoromethylsulfonylimide ([(CF₃SO₂)₂N]⁻) and tetrafluoroborate (BF₄⁻) have attracted attention as anions for most of the ionic liquids so far reported, because of their high electrochemical stabilities and thermal stabilities (Patent Publications 1 and 2).

However, ionic liquids containing these anions suffer from problems such as low conductivity at low temperature, in particular.

Patent Publication 3 discloses boron compounds; however, for example, triethylmethylammonium-CF₃BF₃ manufactured in the Examples has a high melting point of 181°C, and therefore cannot serve as an ionic liquid.

Further, Patent Publication 4 discloses the BF₃CF₃ salt of 1-ethyl-3-methylimidazolium in Example 1.
[Patent Publication 1] Japanese Unexamined Patent Publication No. 2002-099001
[Patent Publication 2] Japanese Unexamined Patent Publication No. 2003-331918
[Patent Publication 3] Japanese Unexamined Patent Publication No. 2002-63934
[Patent Publication 4] Japanese Unexamined Patent Publication No. 2004-123631

### DISCLOSURE OF THE INVENTION

### Problems to be Solved by the Invention

An object of the present invention is to provide ionic liquids with low viscosities, low melting points and high conductivities by improving the anionic and cationic components. The present invention also relates to electric double-layer capacitors and lithium batteries comprising such ionic liquids, especially to lithium secondary batteries.

### Means for Solving the Problems

In view of the aforementioned problems, the present inventors conducted extensive research, and found that an ionic liquid with a low viscosity and low melting point as well as high conductivity at low temperatures can be obtained using at least one anion represented by [BF₃(CₙF₂ₙ₊₁)]⁻ where n represents 1, 2, 3 or 4, or using such an anion together with a salt containing a particular aliphatic or heterocyclic ammonium-based cation.

The present invention provides ionic liquids and a production method therefor as well as electric double-layer capacitors and lithium batteries using such ionic liquids, as itemized below:
1. An ionic liquid comprising:
   at least one anion represented by [BF₃(CₙF₂ₙ₊₁)]⁻ wherein n represents 1, 2, 3 or 4; and
   at least one organic ammonium ion represented by general formula (I):

      [NR¹R²R³R⁴]⁺ (I)
   wherein R¹ to R⁴ are the same or different, each representing an alkyl, fluoroalkyl, alkoxy, polyether, or alkoxyalkyl group, or R¹ and R² taken together with the nitrogen atom may form a pyrrolidine, piperidine, or morpholine ring; provided that R¹ to R⁴ satisfy the conditions (i) through (iii) shown below:
   (i) when R¹ and R² taken together with the nitrogen atom form a pyrrolidine, piperidine, or morpholine ring, either R³ or R⁴ is an alkyl group with 3 or more carbon atoms or alkoxyalkyl group;
   (ii) when R¹ and R² do not form a pyrrolidine, piperidine or morpholine ring, at least one of R¹ to R⁴ is an alkoxy, polyether or alkoxyalkyl group; and
   (iii) when R¹ to R³ are the same or different, each being methyl or ethyl, R⁴ is a C₃₋₁₀ linear or branched alkyl group.
2. An ionic liquid according to item 1, wherein the anion is at least one member selected from the group consisting of [BF₃(CF₃)]⁻, [BF₃(C₂F₅)]⁻ and [BF₃(C₃F₇)]⁻.
3. An ionic liquid according to item 1, wherein R¹, R² and R³ are the same or different, each representing an alkyl group, and R⁴ represents an alkoxyalkyl group.
4. An ionic liquid according to item 1, wherein R¹ and R² taken together with the nitrogen atom form a pyrrolidine, piperidine or morpholine ring; R³ is methyl or ethyl; and R⁴ is an alkyl group with 3 or more carbon atoms or alkoxyalkyl group.
5. An ionic liquid according to item 1, wherein R¹ and R² taken together with the nitrogen atom form a pyrrolidine, piperidine or morpholine ring; R³ is methyl; and R⁴ is an alkyl group with 3 or more carbon atoms or alkoxyalkyl group.
6. An ionic liquid according to item 1, wherein R¹ and R² taken together with the nitrogen atom form a pyrrolidine ring; R³ is methyl; and R⁴ is an alkyl group with 3 or more carbon atoms or alkoxyalkyl group.
7. An electric double-layer capacitor comprising the ionic liquid according to item 1.
8. A lithium battery comprising the ionic liquid according to item 1.
9. A method of producing an ionic liquid comprising mixing a compound containing as an anionic component at least one anion represented by [BF₃(CₙF₂₊₁)]⁻ wherein n represents 1, 2, 3 or 4 with a compound containing as a cationic component at least one organic ammonium ion represented by general formula (I):

   [NR¹R²R³R⁴]⁺ (I)

   wherein R¹ to R⁴ are the same or different, each representing an alkyl, fluoroalkyl, alkoxy, polyether, or alkoxyalkyl group, or R¹ and R² taken together with the nitrogen atom may form a pyrrolidine, piperidine, or morpholine ring; provided that R¹ to R⁴ satisfy the conditions (i) through (iii) shown below:
   (i) when R¹ and R² taken together with the nitrogen atom form a pyrrolidine, piperidine, or morpholine ring, either R³ or R⁴ is an alkyl group with 3 or more carbon atoms or alkoxyalkyl group;
   (ii) when R¹ and R² do not form a pyrrolidine, piperidine or morpholine ring, at least one of R¹ to R⁴ is an alkoxy, polyether or alkoxyalkyl group; and
   (iii) when R¹ to R³ are the same or different, each being methyl or ethyl, R⁴ is a C₃₋₁₀ linear or branched alkyl group.

### EFFECTS OF THE INVENTION

The present invention provides ionic liquids with low viscosities and melting points.

Ionic liquids of the present invention are especially suitable for use in lithium batteries and electric double-layer capacitors. The ionic liquids are also useful in solar cells, electrochemical sensor devices, electrochemical (electrochromic) display devices, and the like.

### BRIEF DESCRIPTION OF THE DRAWINGS

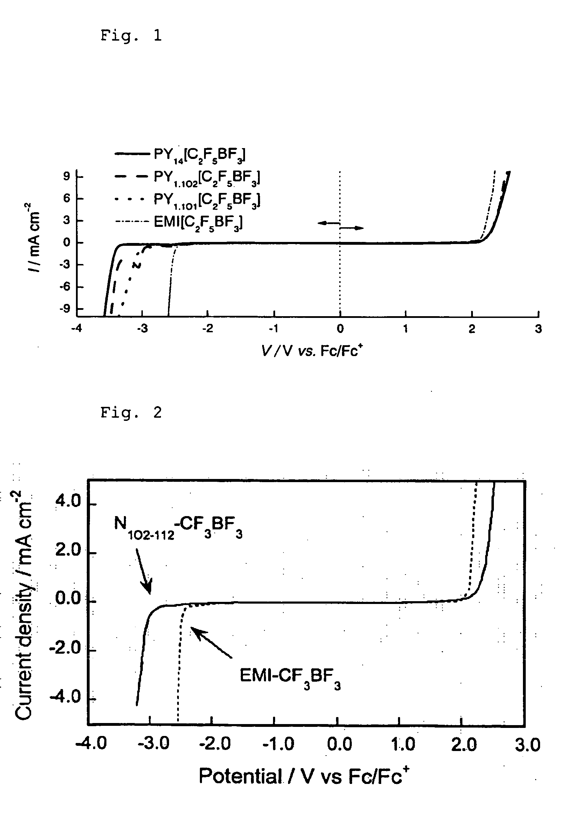

Fig. 1 is a linear sweep voltammogram of ionic liquids measured on a glassy carbon electrode (surface area: 7.85 × 10⁻³ cm⁻²) at the first sweep, wherein the sweep rate is 50 m Vs⁻¹; the counter electrode is a Pt wire; the reference electrode is a platinum wire immersed in EMI-TFSI ionic liquid containing 15 mM iodine and 60 mM tetrapropylammonium iodide dissolved therein in a glass cylinder having its end capped with a glass filter; and the potential (V) for use as the potential reference is the redox potential of the ferrocene (Fc)/ferrocenium (Fc+) redox couple that can be observed when ferrocene is dissolved in each ionic liquid; and
Fig. 2 shows results of linear sweep voltammetry.

### BEST MODE FOR CARRYING OUT THE INVENTION

Ionic liquids for use in the present invention typically have melting points of 150°C or less, preferably 80°C or less, more preferably 60 °C or less, still more preferably 40 °C or less, and even more preferably 25°C or less. For example, ionic liquids with melting points of 150°C or less can be widely used in fuel cells. Ionic liquids for use in energy devices such as solar cells, lithium batteries, capacitors, etc., and electrochemical devices such as electrochromic devices, electrochemical sensors, etc. preferably have melting points of room temperature (25°C) or less, and more preferably 0 °C or less.

The anionic component of the ionic liquid for use in the present invention is at least one member selected from the group consisting of BF₃(CF₃)⁻, [BF₃(C₂F₅)]⁻, [BF₃(C₃F₇)]⁻ (i.e., [BF₃(n-C₃F₇)]⁻ and [BF₃(i-C₃F₇)]⁻), and [BF₃(C₄F₉)]⁻ (i.e., [BF₃(n-C₄F₉)]⁻, [BF₃(i-C₄F₉)]⁻, [BF₃(sec-C₄F₉)]⁻, and [BF₃(tert-C₄F₉)]⁻); and preferably at least one member selected from the group consisting of [BF₃(CF₃)]⁻, [BF₃(C₂F₅)]⁻ , and [BF₃(C₃F₇)]⁻ (i.e., [BF₃(n-C₃F₇)]⁻ and [BF₃)(i-C₃F₇)]⁻); and more preferably [BF₃(CF₃)]⁻ and/or

[BF₃(C₂F₅)]⁻. The above-mentioned anions are known compounds, and are described in, for example, G. A. Molander, B. J. Hoag, Organometallics, 22, (2003), 3313; and Zhi-Bin Zhou, Masayuki Takeda, Makoto Ue, J. Fluorine. Chem., 123 (2003) 127. The ionic liquid of the present invention may comprise a single anionic component, or two or more anionic components to further decrease the melting point.

The ionic liquid can be produced by mixing an organic ammonium compound with a salt of at least one anionic component represented by [BF₃(CₙF₂ₙ₊₁)]⁻ wherein n represents 1, 2, 3 or 4 and a cationic component, such as an alkali metal ion (Na⁺, K⁺, Li⁺, Cs⁺, etc.), an alkaline-earth metal ion (Ca²⁺, Mg²⁺, Ba²⁺, etc.), or H⁺, Bu₃Sn⁺, or the like; and separating an ionic liquid consisting of the organic ammonium ion and [BF₃(CₙF₂ₙ₊₁)]⁻ wherein n represents 1, 2, 3, or 4, or [BF₃(CF=CF₂)]⁻. For example, an ionic liquid consisting of [BF₃(CₙF₂ₙ₊₁)]⁻ wherein n represents 1, 2, 3, or 4 and an organic ammonium ion can be preferably obtained by mixing an (organic ammonium)⁺(OH)⁻ salt with a [BF₃(CₙF₍₂ₙ₊₁)]⁻H⁺ salt, wherein n represents 1, 2, 3, or 4, which is prepared by passing through an ion exchange resin; and removing water. A salt exchange reaction for obtaining an ionic liquid can be carried out by solvent extraction when the desired molten salt is capable of being extracted.

Although a single organic ammonium ion may be used, a combination of two or more organic ammonium ions allows the melting point and viscosity of the ionic liquid to be further decreased.

The anion(s) of the ionic liquid used is at least one member selected from the group consisting of anions represented by [BF₃(CₙF₂ₙ₊₁₎]⁻ wherein n represents 1, 2, 3, or 4, such an anion being the primary component; however, other anions may also be added so long as the resulting salt is an ionic liquid.

Examples of organic ammonium compounds include salts of organic ammonium cations with hydroxide (OH⁻), halogen, nitrate, sulfate, phosphate, perchlorate, methanesulfonate, toluenesulfonate ions, and the like.

The ionic liquid may also be produced using at least one anion selected from the group represented by [BF₃(CₙF₂ₙ₊₁)]⁻ wherein n represents 1, 2, 3 or 4 in the form of, e.g., silver, calcium, barium and/or the like salts, together with an organic ammonium ion, in the form of, e.g., a halide salt, sulfate salt or the like, to form a sparingly soluble salt, such as a silver halide, barium sulfate, calcium sulfate or the like resulting from aforementioned counterions, and removing the formed salt.

Alternatively, the ionic liquid may be prepared by mixing (organic ammonium(s) of general formula (I))⁺(OH)⁻ with at least one member selected from the group consisting of anions represented by [BF₃(CₙF₂ₙ₊₁)]⁻H⁺ wherein n represents 1, 2, 3, or 4.

Examples of alkyl groups include C₁₋₂₀, preferably C_{1-10,} more preferably C₁₋₆, and still more preferably C₁₋₃ linear or branched alkyl groups, such as methyl, ethyl, n-propyl, isopropyl, n-butyl, sec-butyl, isobutyl, t-butyl, pentyl, hexyl, heptyl, octyl, nonyl, decyl, undecyl, dodecyl, tridecyl, tetradecyl, hexadecyl, octadecyl, eicosyl, and the like.

Examples of fluoroalkyl groups include C₁₋₁₀, preferably C₁₋₁₀, more preferably C₁₋₆, and still more preferably C₁₋₃ polyfluoroalkyl and perfluoroalkyl groups wherein at least one of the hydrogen atoms of an above-mentioned alkyl group is substituted with fluorine.

Examples of alkoxy groups include C₁₋₂₀, preferably C₁₋₁₀, more preferably C₁₋₆, and still more preferably C₁₋₃ linear or branched alkoxy groups, wherein an aforementioned alkyl group is attached to oxygen.

The alkoxy and alkyl groups of alkoxyalkyl groups are the same as mentioned above. Examples of alkoxyalkyl groups include C₁₋₂₀, preferably C₁₋₁₀, more preferably C₁₋₆, and still more preferably C₁₋₃ linear or branched alkyl groups substituted with C₁₋₂₀, preferably C₁₋₁₀, more preferably C₁₋₆, and still more preferably C₁₋₃ linear or branched alkoxy groups; such as, preferably -(C₁₋₃ alkylene)-O-(C₁₋₃ alkyl); and more preferably methoxymethyl (CH₂OCH₃), methoxyethyl (CH₂CH₂OCH₃), ethoxymethyl (CH₂OCH₂CH₃), ethoxyethyl (CH₂CH₂OCH₂CH₃), methoxypropyl (CH₂CH₂CH₂OCH₃), ethoxypropyl (CH₂CH₂CH₂OCH₂CH₃), propoxymethyl (CH₂OCH₂CH₂CH₃), propoxyethyl (CH₂CH₂OCH₂CH₂CH₃), isopropoxymethyl (CH₂OCH(CH₃)₂), and isopropoxyethyl (CH₂CH₂OCH(CH₃)₂) groups; and most preferably methoxymethyl (CH₂OCH₃), methoxyethyl (CH₂CH₂OCH₃), ethoxymethyl (CH₂OCH₂CH₃), and ethoxyethyl (CH₂CH₂OCH₂CH₃) groups.

Examples of polyether groups include those represented by -(CH2)ₙ₁-O-(CH₂CH₂O)ₙ₂-(C₁₋₄ alkyl); -(CH₂)ₙ₁-O-(CH₂CH(CH₃)O)ₙ₂-(C₁₋₄ alkyl); or -(CH₂)ₙ₁-O-(CH(CH₃)CH₂O)ₙ₂-(C₁₋₄ alkyl), where n1 is an integer from 1 to 4; n2 is an integer from 1 to 4; and the C₁₋₄ alkyl is, for example, methyl, ethyl, n-propyl, isopropyl, n-butyl, isobutyl, sec-butyl, tert-butyl, or the like.

Alkenyl groups or the aforementioned alkyl groups may have one or more of -O-,-COO- and -CO- interposed between C-C single bonds at any positions to form ether, ester, or ketone structures.

Examples of alkyl groups with 3 or more carbon atoms attached to a pyrrolidine, piperidine, or morpholine ring include C₃₋₂₀, preferably C₃₋₁₀, and more preferably C₃₋₇ linear or branched alkyl groups, such as n-propyl, isopropyl, n-butyl, sec-butyl, isobutyl, t-butyl, pentyl, hexyl, heptyl, octyl, nonyl, decyl, undecyl, dodecyl, tridecyl, tetradecyl, hexadecyl, octadecyl, eicosyl, and the like.

Examples of C₃₋₁₀ alkyl groups represented by R⁴ are C₃₋₁₀, preferably C₄₋₈, and more preferably C₄₋₆ linear or branched alkyl groups, such as n-propyl, isopropyl, n-butyl, sec-butyl, isobutyl, t-butyl, pentyl, hexyl, heptyl, octyl, nonyl, decyl, undecyl, dodecyl, tridecyl, tetradecyl, hexadecyl, octadecyl, eicosyl, and the like.

R¹ and R² taken together with the nitrogen atom to which they are attached may form pyrrolidinium, piperidinium or morpholinium.

Examples of preferable ammonium ions, wherein R¹, R² and R³ are the same or different, each being methyl or ethyl, and wherein R⁴ is a C₃₋₁₀ linear or branched alkyl group, include methyldiethyl(n-, i-, sec-, or tert-)butylammonium (N₁₂₂₄), dimethylethyl(n-, i-, sec-, or tert-)butylammonium (N₁₁₂₄), trimethyl(n-, i-, sec-, or tert-)butylammonium (N₁₁₁₄), triethyl(n-, i-, sec-, or tert-)butylammonium (N₂₂₂₄), methyldiethylhexylammonium (N₁₂₂₆), dimethylethylhexylammonium (N₁₁₂₆), trimethylhexylammonium (N₁₁₁₆), and triethylhexylammonium (N₂₂₂₆).

Examples of organic ammonium ions which can be suitably used in the present invention are illustrated below:

**[Table 1]**

| R¹ | R² | R³ | R⁴ |
|---|---|---|---|
| same or different, each being methyl or ethyl | | | C³⁻¹⁰ alkyl |
| alkyl | alkyl | alkyl | alkoxyalkyl |
| alkyl | alkyl | alkyl | polyether |
| Pyrrolidine ring | | alkyl | alkoxyalkyl |
| Pyrrolidine ring | | alkyl | C₃ or more alkyl |
| Pyrrolidine ring | | alkyl | polyether |
| Pyrrolidine ring | | alkyl | alkoxyalkyl |
| Pyrrolidine ring | | alkyl | C₃ or more alkyl |
| Pyrrolidine ring | | alkyl | polyether |
| morpholine ring | | alkyl | alkoxyalkyl |
| morpholine ring | | alkyl | C₃ or more alkyl |
| morpholine ring | | alkyl | polyether |

Particularly preferable cations in the present invention which are shown by Table 1 and substituted with a lower alkyl group on the nitrogen atom are listed below:
(1) R¹ to R³ are the same or different, each being methyl or ethyl, and R⁴ is a C³⁻¹⁰ alkyl;
(2) R¹ to R³ are the same or different, each being a C₁₋₄ alkyl group, and R⁴ is -(C₁₋₃ alkylene)-O-(C₁₋₃ alkyl) ;
(3) R¹ and R² taken together with the nitrogen atom form a pyrrolidine, piperidine, or morpholine ring; R³ is methyl or ethyl; and R⁴ is a C₁₋₃ alkoxy C₁₋₃ alkyl; and
(4) R¹ and R² taken together with the nitrogen atom form a pyrrolidine, piperidine, or morpholine ring; R³ is methyl or ethyl; and R⁴ is a C₃₋₈ alkyl.

Ionic liquids of the present invention are capable of easily dissolving electrolytes such as lithium salts, and are also incombustible and have low viscosities. Therefore, the ionic liquids can be suitably used as electrolyte solvents for lithium batteries such as lithium secondary batteries, electric double-layer capacitors, solar cells, electrochemical sensor devices, electrochemical (electrochromic) display devices and the like.

### [Examples]

The present invention is described in further detail below with reference to the Examples.

### Reference Example 1: Anion Synthesis

K[CF₃BF₃] was prepared in the manner as described in G. A. Molander, B. J. Hoag, Organometallics, 22, (2003), 3313, and then the K[CF₃BF₃] was subjected to a cation exchange process as described in S. Mori, K. Ida, and M. Ue, US Pat. 4, 892, 944 (1990), thereby yielding aqueous Hₛₒₗᵥ.[CF₃BF₃]solv.

K[C₂F₅BF₃], K[n-C₃F₇BF₃] and K[n-C₄F₉BF₃] were prepared in the manner as described in Zhi-Bin Zhou, Masayuki Takeda, Makoto Ue, J. Fluorine. Chem, 123 (2003) 127, and then the K[C₂F₅BF₃], K[n-C₃F₇BF₃] and K[n-C₄F₉BF₃] were each subjected to a cation exchange process as described in S. Mori, K. Ida, and M. Ue, US Pat. 4, 892, 944. (1990), thereby yielding aqueous ₛₒₗᵥ[n-C₂F₅BF₃]ₛₒₗᵥ, Hₛₒₗᵥ[n-C₃F₇BF₃]ₛₒₗᵥ and Hₛₒₗᵥ[n-C₄F₉BF₃]ₛₒₗᵥ, respectively.

### Reference Example 2: Cation Synthesis

### (1) Synthesis of diethylmethylmethoxyethylammonium chloride (C3: N₁₀₂₁₂₂⁺Cl⁻)

An amine (diethylmethylamine) and an equimolar amount of a halogen-substituted ether compound (methoxyethylchloride) as starting materials were mixed in acetonitrile, and then the mixture was reacted for 12 to 72 hours by heating in an autoclave under mild conditions. After the reaction, the quaternary ammonium salt product was recrystallized in an appropriate solvent, and the formation of diethylmethylmethoxyethylammonium chloride (N₁₀₂₁₂₂⁺Cl⁻) was confirmed by NMR.

The halide thus obtained was converted to the hydroxide (N₁₀₂₁₂₂⁺(OH⁻) with an anion exchange resin.

### (2) Synthesis of trimethylmethoxyethylammonium bromide (C1: N₁₀₂₁₁₁⁺Br⁻); dimethylethylmethoxyethylammonium bromide (C2: N₁₀₂₁₁₂⁺Br⁻); and triethylmethoxyethylammonium bromide (C4: N₁₀₂₂₂₂⁺Br⁻)

CH₃OCH₂CH₂Br and an equimolar amount of an amine (one each of triethylamine, dimethylethylamine or triethylamine) as starting materials were mixed in anhydrous acetone, and then each mixture was reacted for 12 to 72 hours by heating in an autoclave under mild conditions. After the reaction, each quaternary ammonium salt product was recrystallized in acetone, and the formation of trimethylmethoxyethylammonium bromide (N₁₀₂₁₁₁⁺Br⁻), dimethylethylmethoxyethylammonium bromide (N₁₀₂₁₁₂⁺Br⁻) and triethylmethoxyethylammonium bromide (N₁₀₂₂₂₂⁺Br⁻) was confirmed by NMR.

The bromides thus obtained were converted to the hydroxides (N₁₀₂₁₁₁⁺OH⁻, N₁₀₂₁₁₂⁺OH⁻ and N₁₀₂₂₂₂⁺OH⁻, respectively) with an anion exchange resin.

### (3) Synthesis of methylmethoxyethylpiperidinium bromide (C5: Pi_{102.1}⁺Br⁻); methylmethoxyethylpyrrolidinium bromide (C6: Py_{102.1}⁺Br⁻); ethyldimethylmethoxymethylammonium bromide (C7: N₁₀₂.₁₁₂⁺Br⁻); butyldimethylmethylammonium bromide (C8: N₁₂₂₄⁺Br⁻); methylmethoxymethylpyrrolidinium bromide (C9: Py_{101.1}⁺Br⁻); methylbutylmorpholinium bromide (C10: Mor₁₄⁺Br⁻); and methylmethoxyethylmorpholinium bromide (C11: Mor_{1.102}⁺Br⁻)

C5 (Pi_{102.1}⁺Br⁻), C6 (Py_{102.1}⁺Br⁻) and C11 (Mor_{1.102}⁺Br⁻) were synthesized in a similar manner as in synthesis (2) above, except for using N-methylpyrrolidine, N-methylpiperidine and N-methylmorpholine instead of the amines (triethylamine, dimethylethyl amine and triethylamine).

In addition, C7 (N_{102.112}⁺Br⁻), C8 (N₁₂₂₄⁺Br⁻), C9 (Py_{101.1}⁺Br⁻) and C10 (Mor₁₄⁺Br⁻) were synthesized in a similar manner as in synthesis (2) above, except for using dimethylethylamine, methyldiethylamine, methylpyrrolidine or N-methylmorpholine as the amine; and using CH₃CH₂CH₂CH₂Br or CH₃OCH₂Br as the bromide.

The bromides thus obtained were converted to the hydroxides (C5: Pi_{102.1}⁺OH⁻; C6: Py₁₀₂.₁⁺OH⁻; C7: N₁₀₂.₁₁₂⁺OH⁻; C8: N₁₂₂₄⁺OH⁻; C9: Py_{101.1}⁺OH⁻; C10: Mor₁₄⁺OH⁻; and C11: Mor₁.₁₀₂⁺OH⁻, respectively) with an anion exchange resin.

The structural formulae of ammonium ions C1 through C11 are shown below:

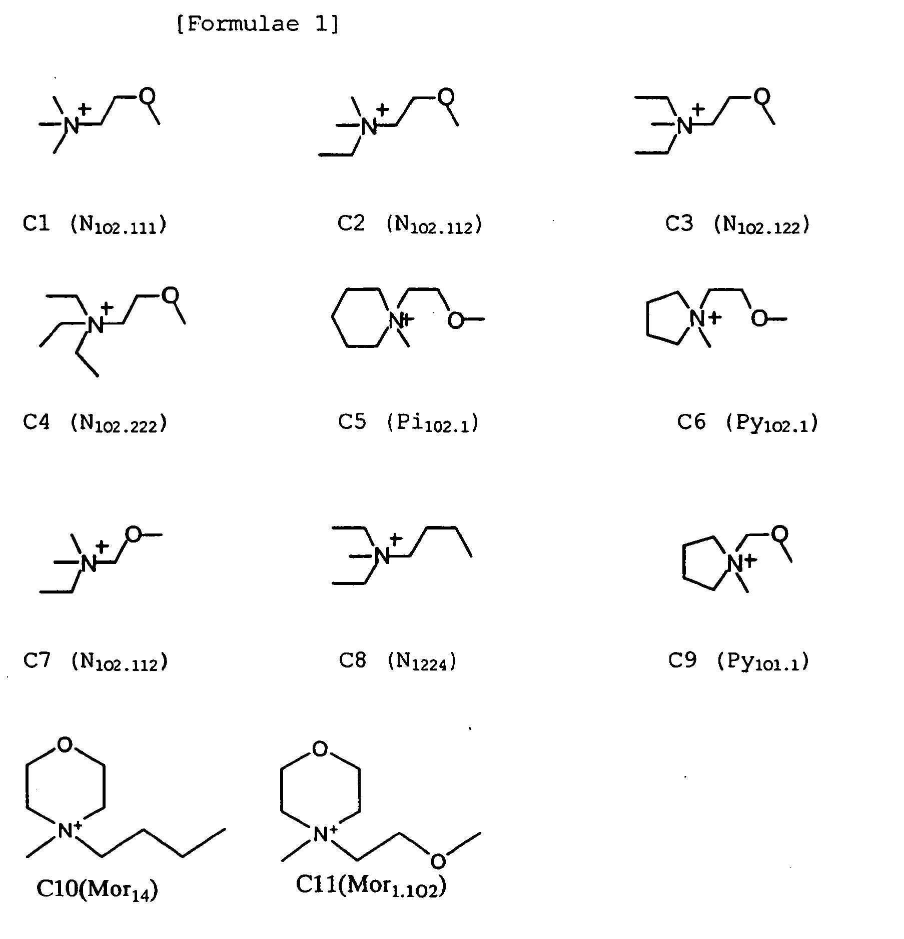

### (4) Synthesis of pyrrolidine-based quaternary ammonium salts

Cations shown below were synthesized in a similar manner as in synthesis (2), except for using N-methylpyrrolidine instead of the amines (triethylamine, dimethylethyl amine and triethylamine), and using CH₃(CH₂)ₚBr where p is an integer from 0 to 6, CH₃OCH₂Br, CH₃OCH₂CH₂Br, CH₃CH₂OCH₂CH₂Br, or CH₃O(CH₂CH₂)₂OCH₂CH₂Br as the bromide, and the bromides obtained were then converted to the hydroxides with an anion exchange resin. The cations are shown below along with their abbreviations:

**[Table 2]**

| | | | |
|---|---|---|---|
| 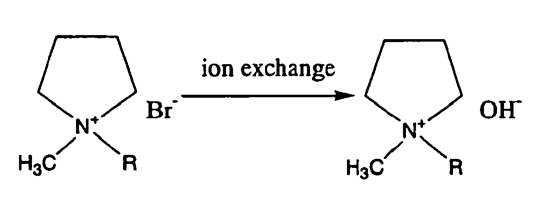 | | | |

| R | Cation | R | Cation |
|---|---|---|---|
| CH₃ | Py₁₁ | n-C₆H₁₃ | Py₁₆ |
| C₂H₅ | Py₁₂ | n-C₇H₁₅ | Py₁₇ |
| n-C₃H₇ | Py₁₃ | C₂H₅O (CH₂) | Py_{1.202} |
| n-C₄H₉ | Py₁₄ | CH₃O(CH₂)O(CH₂)₂ | Py_{1.10202} |
| n-C₅H₁₁ | Py₁₅ | | |

### (5) Known ammonium compounds

In addition to the above, methyltriethylammonium hydroxide (N₁₂₂₂·OH⁻) and tetraethylammonium hydroxide (N₂₂₂₂·OH⁻) were prepared by a known process.

### Example 1: Preparation of Ionic Liquids

An aqueous solution (50 mmol) of any one of the anions (H_{solv.}[CF₃BF₃]_{solv,} Hₛₒₗᵥ[n-C₂F₅BF₃]ₛₒₗᵥ, Hₛₒₗᵥ[n-C₃-F₇BF₃]ₛₒₗᵥ and Hₛₒₗᵥ[n-C₄F₉BF₃]ₛₒₗᵥ) obtained in Reference Example 1 was filtered before use, and then the filtrate was neutralized by an equimolar amount of any one of the hydroxides of ammonium cations obtained in Reference Example 2. The ionic liquid was concentrated to about 20 ml under reduced pressure at 30 to 40 °C, and then the bottom layer was separated, followed by washing with deionized water (10 ml) and toluene (20 ml × 2). The resulting ionic liquid bottom layer was dried under vacuum (0.03 mmHg) at 60 °C for 12 hours, so as to yield the target ionic liquid.

Tables 3 to 5 below show the combinations of the anions and cations along with their physical values.

In addition, data such as NMR (¹H, ¹¹B and ¹⁹F), elemental analysis and the like on some of the ionic liquids obtained are presented below:

N₁₀₂.₁₂₂[BF₄]

¹H NMR (399.65 MHz/acetone-d₆, δ ppm relative to internal TMS): 1.39 (t, *J* = 7.2Hz, NCH₂C*H*₃), 3.18 (s, NC*H*₃), 3.38 (s, OCH₃), 3.58 (q, *J* = 7.3Hz, NC*H₂*CH₃), 3.67 (t, *J* = 4.8Hz, OCH₂C*H₂*N), 3.88 (s, OC*H₂*CH₂N).

Anal. Calc. for C₈H₂₀BF₄NO: C, 41.2; H, 8.7; N, 6.0. Found: C, 41.3; H, 8.5; N, 5.9%. 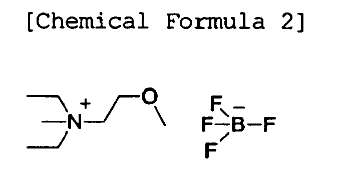 N_{102.122}[n-C₂F₅BF₃]

¹H NMR: (399.65 MHz/acetone-d₆, δ ppm relative to internal TMS): 1.41 (t, *J* = 7.2 Hz, NCH₂C*H*₃), 3.19 (s, NC*H*₃), 3.39 (s, OC*H*₃), 3.59 (q, *J* = 7.2 Hz, NC*H*₂CH₃), 3.67 (t, *J* = 4.8 Hz, OCH₂C*H₂*N), 3.91 (s, OC*H₂*H₂N).

¹⁹F NMR (376.05 MHz/acetone-d₆, δ ppm relative to external CCl₃F): -83.0 (s, CF₃), 135.8 (q, ²*J*_{BF} = 20.3Hz, CF₂), -152.8 (q, ¹*J*_{BF} = 40.7 Hz, BF₃).

¹¹B NMR (128.15 MHz/acetone-d₆, δ ppm relative to external BF₃.Et₂O): 0.149 (qt, ¹*J*BF = 40.8 Hz, ²*J*_{BF} = 19.1 Hz).

Anal. Calc. for C₁₀H₂₀BF₈NO: C, 36.1; H, 6.1; N, 4.2. Found: C, 36.4; H, 4.2; H, 6.0; N, 4.5%. 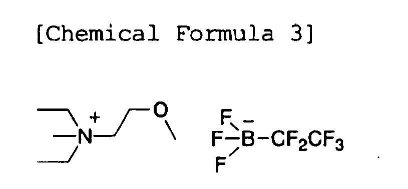 N₁₀₂.₁₂₂[n-C₃F₇BF₃]

¹H NMR (399.65 MHz/acetone-d₆, δ ppm relative to internal TMS) : 1. 41 (t, *J* = 7.3 Hz, NCH₂C*H*₃), 3.20 (s, NC*H*₃), 3.38 (s, OC*H*₃), 3.59 (q, *J* = 7.2 Hz, NC*H*₂CH₃), 3.67 (t, *J* = 4.8 Hz, OCH₂*CH₂*N), 3.91 (s, OC*H₂*CH₂N)

¹⁹F NMR (376.05 MHz/acetone-d₆, δ ppm relative to external CCl₃F): -80.3 (s, CF₃), -127.5 (s, CF₃CF₂), 133.7 (s, CF₂B), -152.3 (q, ¹*J*_{BF} = 38.7 Hz, BF₃).

¹¹B NMR (128.15 MHz/acetone-d₆, δ ppm relative to external BF₃.Et₂O): 0.246 (qt, ¹*J*BF = 40.6 Hz, ²*J*_{BF}=19.0 Hz).

Anal. Calc. for C₁₁H₂₀BF₁₀NO: C, 34.5; H, 5.3; N, 3.7. Found: C, 34.7; H, 5.2; N, 3.7%. 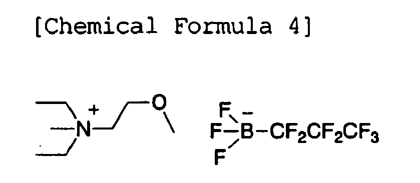 N₁₀₂.₁₂₂[n-C4F₉BF₃]

¹H NMR (399.65 MHz/acetone-d₆, δ ppm relative to internal TMS): 1.41 (m, NCH₂C*H₃*), 3.21 (m, NC*H*₃), 3.38 (m, OC*H*₃), 3.60 (q, J = 7.2 Hz, NC*H*₂CH₃), 3.67 (t, *J* = 4.8 Hz, OCH₂C*H₂*N), 3.91 (s, OC*H*₂CH₂N).

¹⁹F NMR (376.05 MHz/acetone-d₆, δ ppm relative to external CCl₃F): -80.9 (s, CF₃), -123. 8 (s, CF₃C*F₂*), 125.8 (s, CF₃CF₂C*F₂*), 133.1 (s, CF₂B), -152.3 (q, ¹*J*_{BF} = 38.7 Hz, BF₃).

¹¹B NMR (128.15 MHz/acetone-d₆, δ ppm relative to external BF₃.Et₂O) : 0.233 (qt, ¹*J*_{BF} = 40.3 Hz, ²*J*_{BF} = 19.0 Hz).

Anal. Calc. for C₁₂H₂₀BF₁₂NO: C, 33.3; H, 4.7; N, 3.2. Found: C, 33.6; H, 4.6; N, 3.4%. 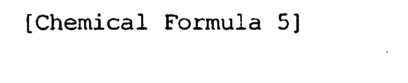 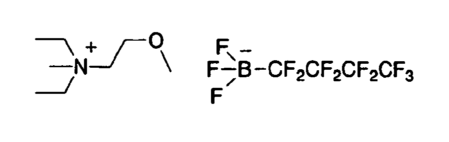 N_{102.111} [C₂F₅BF₃]

Elemental Analysis Calc. (Found): C, 31.5 (31.2); H, 5.3 (5.2); N, 4.6 (4.6)%.

¹H NMR: 3.37 (s, 3 × 3H), 3.40 (s, 3H), 3.76 (s, 2H), 3.94 (s, 2H).

¹⁹F NMR: -83.0 (s, CF₃), -135.8 (q, ²*J*_{BF} = 19. 3 Hz, CF₂), -153.0 (q, ¹*J*_{BF} = 39.6 Hz, BF₃).

N_{102.112}[C₂F₅BF₃]

Elemental Analysis Calc. (Found): C, 33.9 (33.7); H, 5.7 (5.6); N, 4.4 (4.3)%.

¹H NMR: 1.45 (t, *J* = 7.2 Hz, 3H), 3.28 (s, 2 × 3H), 3.39 (s, 3H), 3.64 (q, *J* = 7.2 Hz, 2H), 3.71 (t, *J* = 4.8 Hz, 2H), 3.92 (s, 2H).

¹⁹F NMR: -83.0 (s, CF₃), -135.8 (q, ²*J*_{BF} = 19.3 Hz, CF₂), -152.7 (q, ¹*J*_{BF} = 40.7 Hz, BF₃).

N_{102.122}[C₂FsBF₃]

Elemental Analysis Calc. (Found): C, 36.1 (35.8); H, 6.1 (5.9); N, 4.2 (4.1)%.

¹H NMR: 1.41 (t, *J* = 7.2 Hz, 2 × 3H), 3.19 (s, 3H), 3.39 (s, 3H), 3.59 (q, *J* = 7.2 Hz, 2 × 2H), 3.67 (t, *J* = 4.8 Hz, 2H), 3.91 (s, 2H).

¹⁹F NMR: -83.0 (s, CF₃), -135.8 (q, ²*J*_{BF} = 20.3 Hz, CF₂), -152.8 (q, ¹*J*_{BF} = 40.7 Hz, BF₃).

N₁₀₂.₂₂₂[C₂F₅BF₃]

Elemental Analysis Calc. (Found): C, 38.1 (38.1); H, 6.4 (6.4); N, 4.0 (4.0)%.

¹H NMR: 1.37 (t, *J* = 7.2 Hz, 3 × 3H), 3.38 (s, 3H), 3.56 (q, *J*= 7.2 Hz, 3 × 2H), 3.63 (t, *J*= 4.8 Hz, 2H), 3.87 (s, 2H).

¹⁹F NMR: -83.0 (s, CF₃), -135.8 (q, ²*J*_{BF} = 19.4 Hz, CF₂), -153.0 (q, ¹*J*_{BF} = 39.7 Hz, BF₃).

DMI [CF₃BF₃]

Elemental Analysis Anal. Calc. (Found): C, 30.8 (30.5); H, 3.9 (4.0); N, 12.0 (11.9)%.

¹H NMR: 4.02 (s, 2 × 3H, NC*H*₃), 7.66 (m, 2H, N-C*H*=C*H*-N), 8.89 (s, 1H, N-C*H*-N).

PMI [CF₃BF₃]

Elemental Analysis Calc. (Found): C, 36.7 (36.5); H, 5.0 (5.1); N, 10.7 (10.8)%.

¹H NMR: 0.96 (t, *J* = 7.2 Hz, 3H, CCH₃), 1.98 (m, 2H, CH₃C*H₂*-), 4.06 (s, 3H, N-C*H₃*), 4.32 (q, *J* = 7.3 Hz, 2H, NC*H*₂-), 7.71 and 7.75 (s, 2H, N-C*H*=C*H*-N), 8.99 (s, 1H, N-C*H*-N).

BMI [CF₃BF₃]

Elemental Analysis Calc. (Found): C, 39.2 (38.9); H, 5.5 (5.8); N, 10.2 (10.2)%.

¹H NMR: 0.95 (t, *J* = 7.2 Hz, 3H, CC*H*₃), 1.40 (m, 2H, CH₃C*H₂-*), 1.93 (m, 2H, CH₃CC*H₂*-), 4.04 (s, NC*H*₃), 4.35 (q, *J* = 7.3 Hz, 2H, NC*H*₂-), 7.68 and 7.74 (s, 2H, N-C*H*=C*H*-N), 8.95(s, 1H, N-C*H*-N).

HMI [CF₃BF₃]

Elemental Analysis Calc. (Found): C, 43.5 (43.2); H, 6.3 (6.0); N, 9.2 (9.3)%.

¹H NMR: 0.87 (t, *J* = 7.0 Hz, 3H, CC*H*₃), 1.34 (m, 3 × 2H, CH₃(C*H₂*)₃-), 1.95 (m, 2H, NCH₂C*H₂*-), 4.04 (s, 3H, NC*H*₃), 4.35 (t, *J* = 7.2 Hz, 2H, NC*H*₂-), 7.69 and 7.75 (s, 2H, N-C*H*=C*H*-N), 8.97 (s, 1H, N-C*H*-N).

In Tables 3 to 5, d = density at 25 °C; Tg = glass transition temperature (on heating); Tc = crystallization temperature (on heating); Tm = melting point (on heating); η = viscosity at 25 °C; κ = conductivity at 25 °C; and Nd = not detected.

**[Table 3]**

| | | | | | | |
|---|---|---|---|---|---|---|
| Physicochemical Properties of Ionic liquids Containing Ammonium Cations | | | | | | |

| salt | Tg /°C | Tc /°C | Tm /°C | Td /°C | η/m Pas | κ/m Scm⁻¹ |
|---|---|---|---|---|---|---|
| N_{102.222} [BF₄] | Nd | Nd | 56 | 372 | Solid | Solid |
| N_{102.122} [BF₄] | -95 | -51 | 8 | 372 | 426 | 1.3 |
| N₁₀₂.₁₁₂ [BF₄] | -97 | -26 | 4 | 377 | 335 | 1.7 |
| N_{102.111} [BF₄] | Nd | Nd | 54 | 376 | Solid | Solid |
| N₁₂₂₄ [BF₄] | Nd | Nd | 165 | 392 | Solid | Solid |
| N_{101.112} [CF₃BF₃] | Nd | Nd | 30 | 173 | Solid | Solid |
| N_{102.122} [CF₃BF₃] | Nd | Nd | -22 | 174 | 108 | 3.0 |
| N_{102.222} [CF₃BF₃] | Nd | Nd | 10 | 210 | 151 | 2.0 |
| N_{102.112} [CF₃BF₃] | Nd | Nd | 8 | 163 | 97 | 2.5 |
| Py_{102.1} [CF₃BF₃] | Nd | Nd | -15 | 232 | 87 | 4.3 |
| Pi_{102.1} [CF₃BF₃] | Nd | Nd | -16 | 234 | 203 | 1.8 |
| N₁₂₂₄ [CF₃BF₃] | Nd | Nd | -3 | 212 | 210 | 2.1 |

**[Table 4]**

| salt | Tg /°C | Tc /°C | Tm /°C | Td /°C | η/m Pas | κ/m Scm⁻¹ |
|---|---|---|---|---|---|---|
| N_{102.122} [C₂F₅BF₃] | -113 | Nd | Nd | 322 | 68 | 3.2 |
| N_{102.122} [n-C₃F₇BF₃] | -112 | Nd | Nd | 275 | 88 | 1. 9 |
| N_{102.122} [n-C₄F₉BF₃] | -108 | Nd | Nd | 287 | 118 | 1.3 |
| N_{102.111} [C₂F₅BF₃] | Nd | Nd | 30 | 326 | Solid | Solid |
| N_{102.112} [C₂F₅BF₃] | -117 | -76 | -33 | 307 | 58 | 3.8 |
| N_{102.222} [C₂F₅BF₃] | -98 | -63 | 3 | 345 | 87 | 2.4 |
| N_{101.112} [C₂F₅BF₃] | Nd | Nd | 11 | 287 | 44 | 5. 4 |
| Py_{101.1} [C₂F₅BF₃] | Nd | Nd | 26 | 299 | 37 | 6.8 |
| Py_{102.1} [C₂F₅BF₃] | Nd | Nd | -3 | 289 | 52 | 4.5 |
| Pi_{102.1} [C₂F₅BF₃] | Nd | Nd | -17 | 301 | 112 | 2.2 |
| N₁₂₂₄ [C₂F₅BF₃] | Nd | Nd | 15 | 320 | 104 | 2.3 |
| N_{102.112} [n-C₃F₇BF₃] | -113 | Nd | Nd | 291 | 70 | 2.6 |
| N_{102.222} [n-C₃F₇BF₃] | Nd | Nd | 6 | 351 | 91 | 1.8 |
| N_{102.111} [n-C₃F₇BF₃] | Nd | Nd | 23 | 284 | 76 | 2.5 |
| Py_{102.1} [n-C₃F₇BF₃] | Nd | Nd | 5 | 283 | 62 | 3.3 |
| Pi_{102.1} [n-C₃F₇BF₃] | Nd | Nd | 21 | 297 | 187 | 0.93 |
| N_{102.222} [n-C₄FgBF₃] | Nd | Nd | 11 | 305 | 135 | 1.1 |
| N_{102.112} [n-C₄F₉BF₃] | -110 | -56 | -28 | 283 | 102 | 1.5 |
| Py_{102.1} [n-C₄F₉BF₃] | -100 | -63 | -13 | 284 | 84 | 2.1 |
| Pi_{102.1} [n-C₄F₉BF₃] | -91 | -62 | -7 | 298 | 131 | 1. 5 |
| Py₁₁ [C₂F₅BF₃] | Nd | Nd | >150 | 325 | Solid | Solid |
| Py₁₂ [C₂F₅BF₃] | Nd | Nd | >150 | 307 | Solid | Solid |
| Py₁₃ [C₂F₅BF₃] | Nd | Nd | 63 | 312 | Solid | Solid |
| Py₁₄ [C₂F₅BF₃] | Nd | Nd | 22 | 311 | 71 | 3.5 |
| Py₁₅ [C₂F₅BF₃] | Nd | Nd | 36 | 307 | Solid | Solid |
| Py₁₆ [C₂F₅BF₃] | Nd | Nd | 58 | 307 | Solid | Solid |
| Py₁₇ [C₂F₅BF₃] | Nd | Nd | 52 | 311 | Solid | Solid |
| Py_{1.101} [C₂F₅BF₃] | Nd | Nd | 26 | 299 | 37 | 6.8 |
| Py_{1.102} [C₂F₅BF₃] | Nd | Nd | -3 | 289 | 52 | 4.5 |
| Py_{1.202} [C₂F₅BF₃] | -108 | Nd | -6 | 290 | 49 | 3.7 |
| Py_{1.10202} [C₂F₅BF₃] | -98 | Nd | Nd | 297 | 54 | 3.0 |

**[Table 5]**

| | | | | | | |
|---|---|---|---|---|---|---|
| Ionic liquids Containing Morpholinium Cations | | | | | | |

| salt | Tg/°C | Tc/°C | Tm/°C | Td/°C | η/m Pas | κ/m Scm⁻¹ |
|---|---|---|---|---|---|---|
| Mor₁₄ [CF₃BF₃] | -73 | Nd | Nd | 181 | 1035 | 0.37 |
| Mor₁₄ [C₂F₅BF₃] | -72 | Nd | Nd | 303 | 466 | 0.51 |
| Mor₁₄ [n-C₃F₇BF₃] | Nd | Nd | 69 | 317 | Solid | Solid |
| Mor₁₄ [n-C₄F₉BF₃] | Nd | Nd | 77 | 300 | Solid | Solid |
| Mor₁₄ [BF₄] | Nd | Nd | 66 | 382 | Solid | Solid |
| Mor_{1.102} [CF₃BF₃] | Nd | -42 | 0 | 232 | 471 | 0.68 |
| Mor_{1.102} [C₂F₅BF₃] | -78 | Nd | Nd | 306 | 260 | 0.85 |
| Mor_{1.102} [n-C₃F₇BF₃] | -75 | Nd | Nd | 302 | 377 | 0.51 |
| Mor_{1.102} [n-C₄F₉BF₃] | Nd | Nd | 13 0 | 291 | Solid | Solid |
| Mor_{1.102}[BF₄] | -58 | 2.1 | 85 | 365 | Solid | Solid |

### Test Example 1: Measurement of Physical Values

Fig. 1 shows a linear sweep voltammogram of the [C₂F₅BF₃] salt.

Fig. 2 shows the results of linear sweep voltammetry performed on N₁₀₂₁₁₂[CF₃BF₃] and EMI [CF₃BF₃] at room temperature in a glove box (O₂ and water < 5 ppm) filled with argon for the evaluation of electrochemical stability (working electrode: glassy carbon; counter electrode: platinum; reference electrode: a platinum wire immersed in iodine redox-containing EMI-TFSI. Calculated using the redox potential of ferrocene in the ionic liquid as an internal standard. Measured by ALS, model 660 electrochemical analyzer).

The results of Fig. 2 show that the reduction and oxidization potentials of N₁₀₂₁₁₂[CF₃BF₃] shifted to more negative and positive potentials, respectively, than those of EMI[CF₃BF₃]; therefore the electrochemical stability of N₁₀₁₁₂ [CF₃BF₃] is enhanced.

The results presented above demonstrate that the ionic liquid N₁₀₂₁₁₂ [CF₃BF₃] of the present invention has a high conductivity and low melting point, hence exhibiting superior properties as a solvent for electrochemical devices and organic reactions.

### Comparative Examples 1 through 4

Four compounds shown below which are disclosed in the specification of Patent Publication 3 (Japanese Unexamined Patent Publication 2002-63947) and in Table 1 were synthesized, and the melting points of these compounds were measured. The results are shown below:

**[Table 6]**

| Salt | Melting point |
|---|---|
| Triethylmethylammonium (TEMA) [CF₃BF₃] | 181 °C |
| Tetraethylammonium [CF₃BF₃] | 237 °C (decomposition) |
| N,N'-dimethylpyrrolidinium [CF₃BF₃] | m. p.: 325 °C (decomposition) |
| N-methyl-N'-ethylpyrrolidinium [CF₃BF₃] | m. p.: 280 °C (decomposition) |

A comparison with the results above show that the melting points of ionic liquids greatly vary with slight differences in the structure of the ammonium cation.

## Claims

1. An ionic liquid comprising at least one member selected from the group consisting of

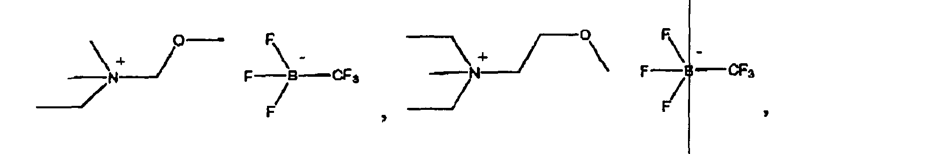

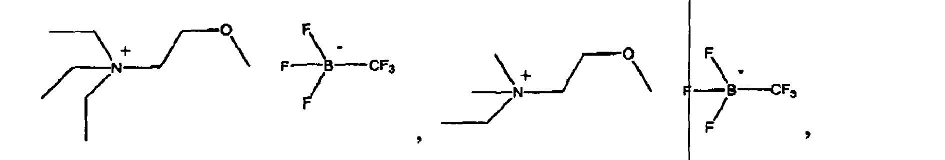

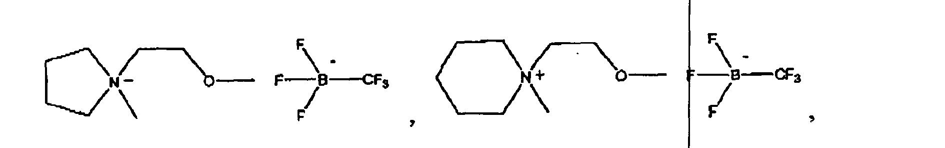

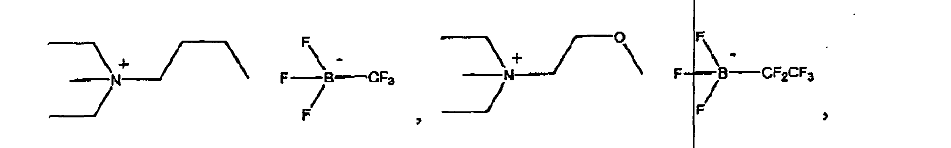

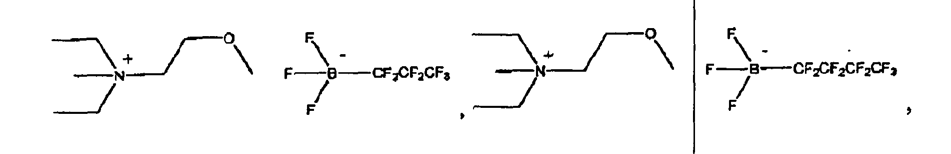

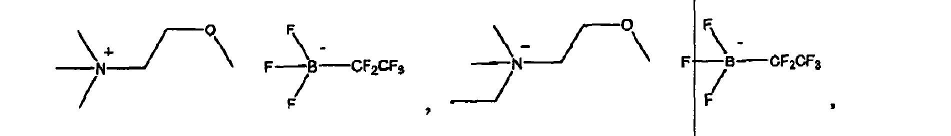

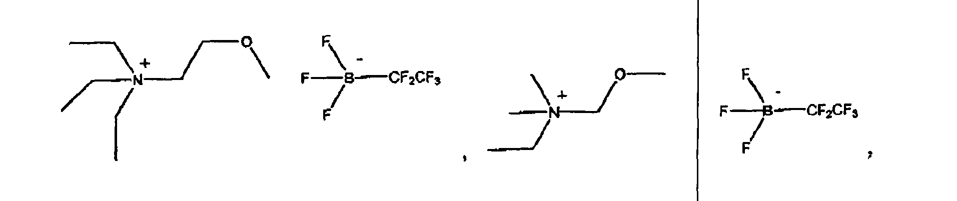

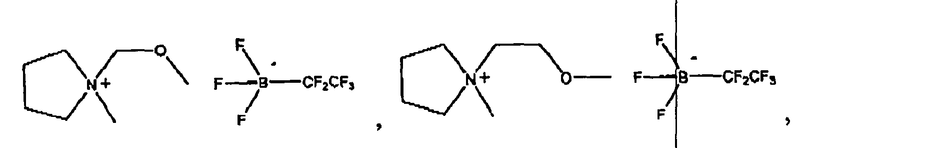

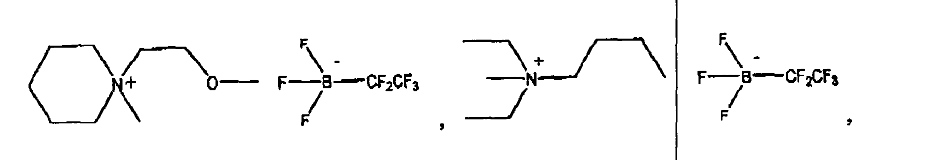

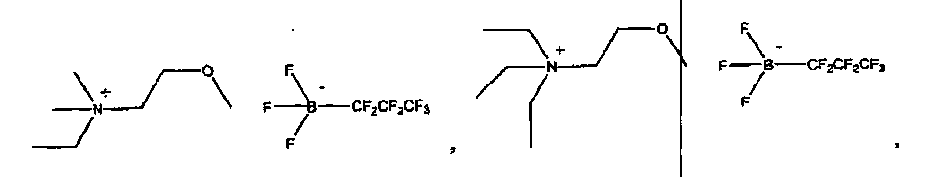

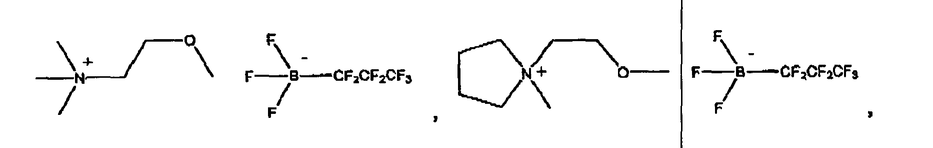

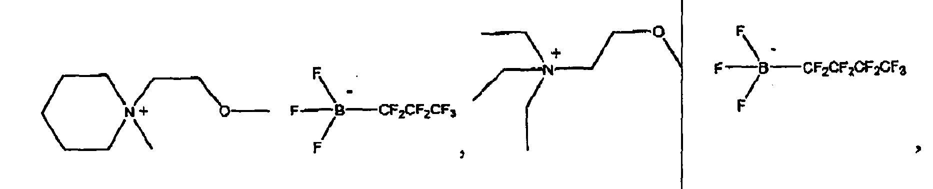

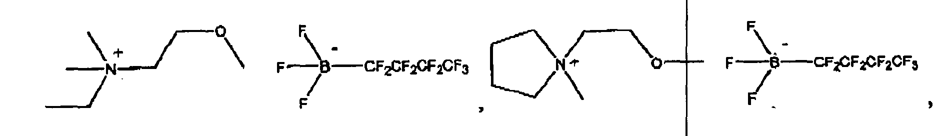

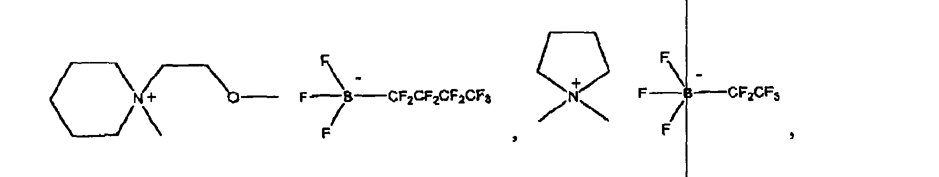

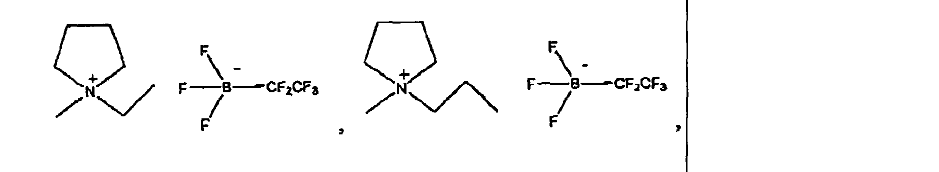

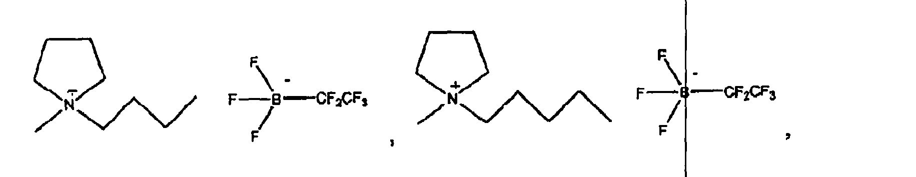

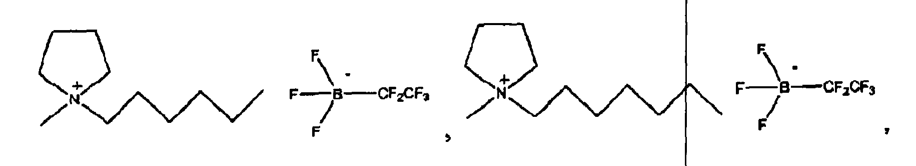

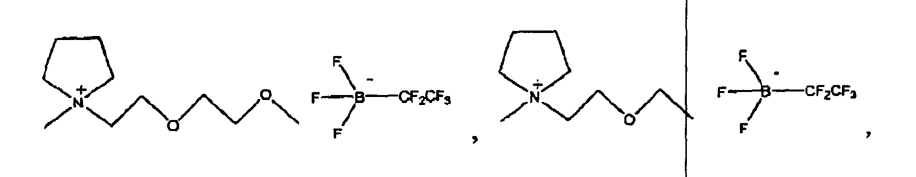

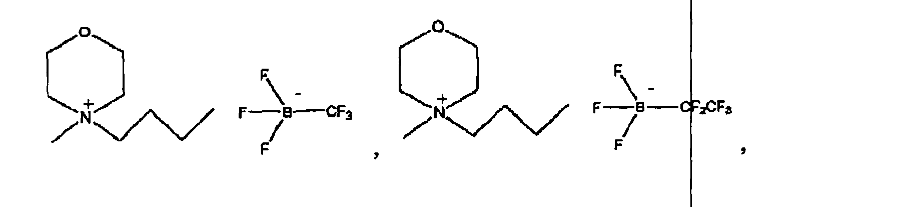

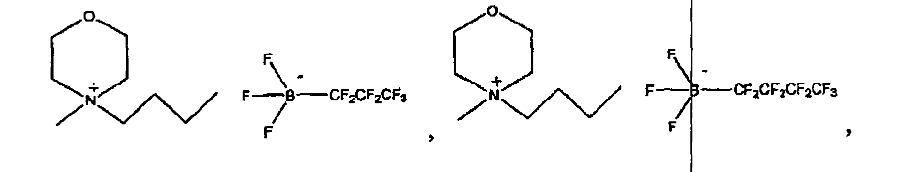

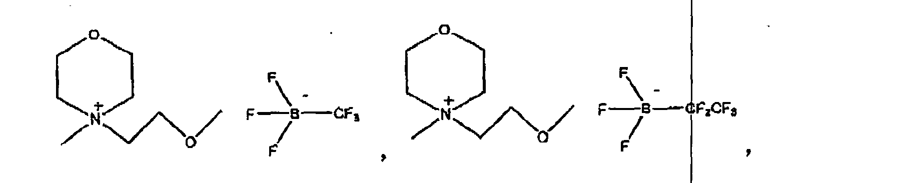

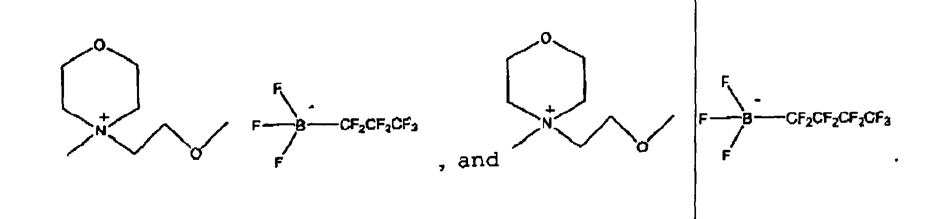

2. An electric double-layer capacitor comprising the ionic liquid according to claim 1.

3. A lithium battery comprising the ionic liquid according to claim 1.

4. A method of producing an ionic liquid according to Claim 1 comprising mixing a compound containing as an anionic component at least one anion represented by [BF₃(CₙF₂ₙ₊₁)]⁻ wherein n represents 1, 2, 3 or 4 with a compound containing as a cationic component at least cne organic ammonium ion are the same or different, each selected from the group consisting of 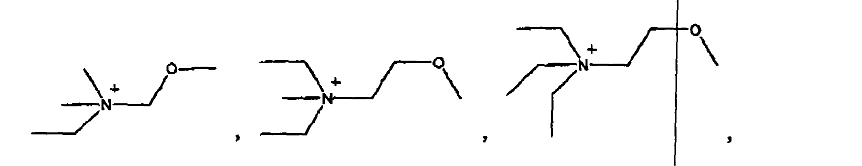 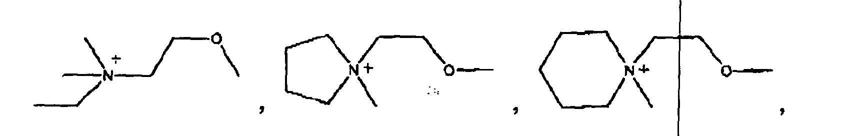 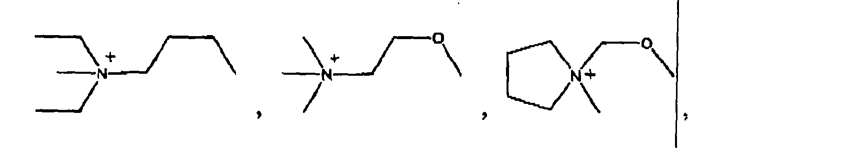 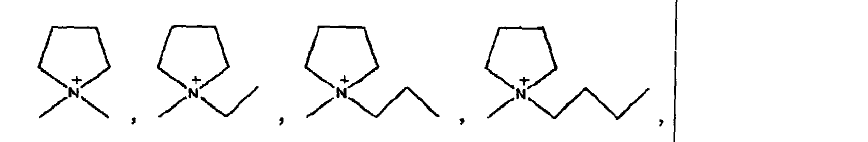 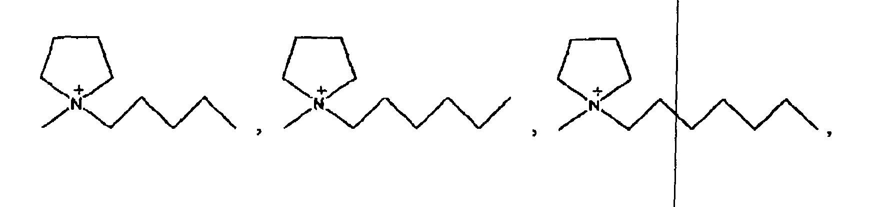 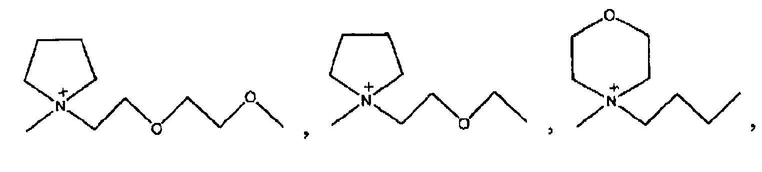 and

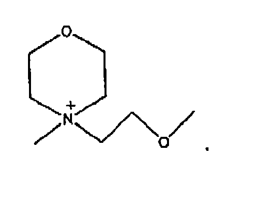

## Patentansprüche

1. Ionische Flüssigkeit umfassend mindestens einen Bestandteil ausgewählt aus der Gruppe bestehend aus

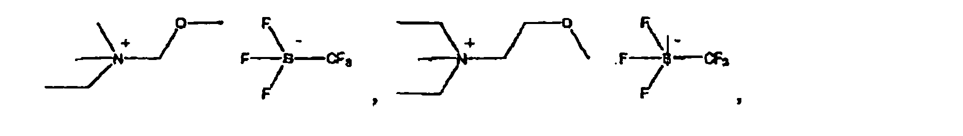

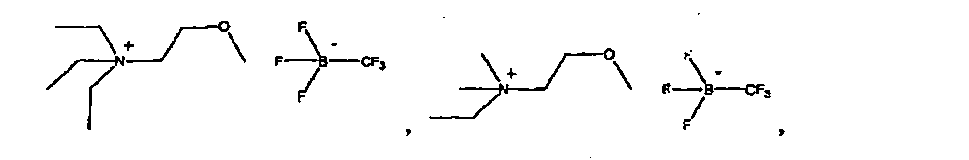

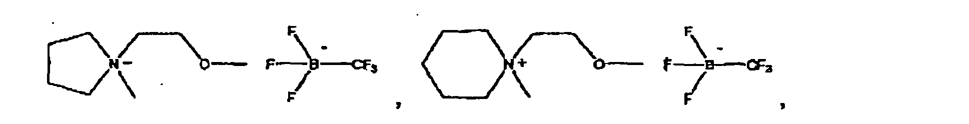

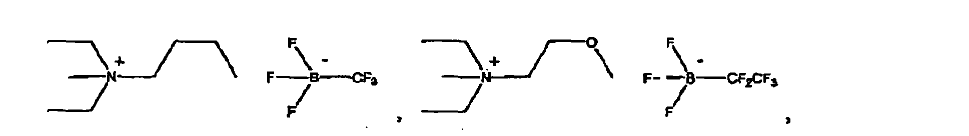

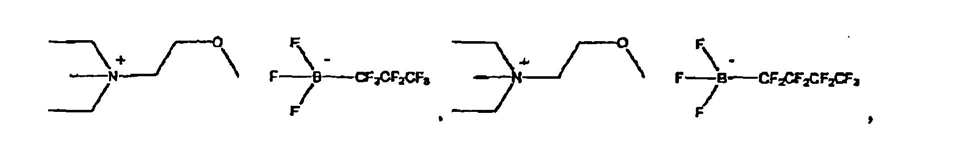

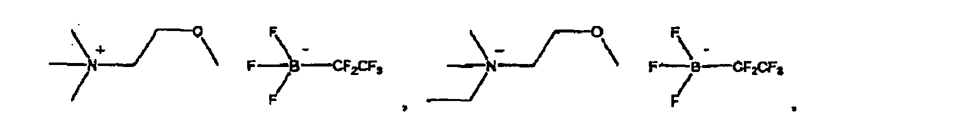

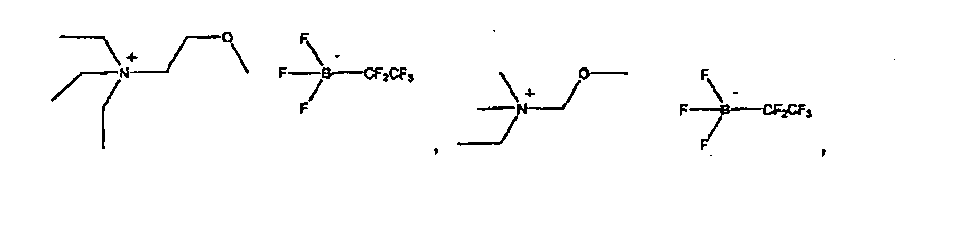

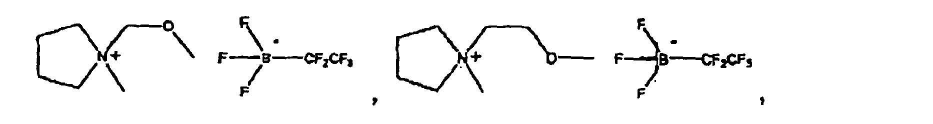

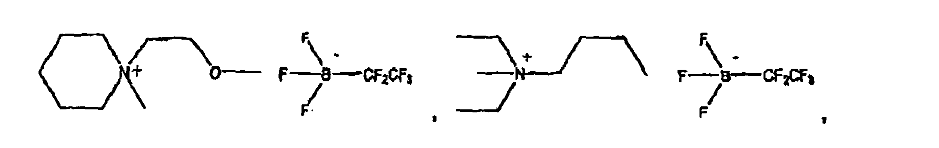

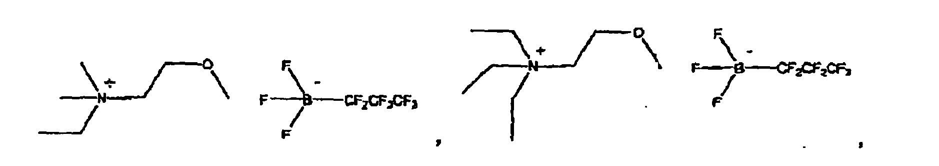

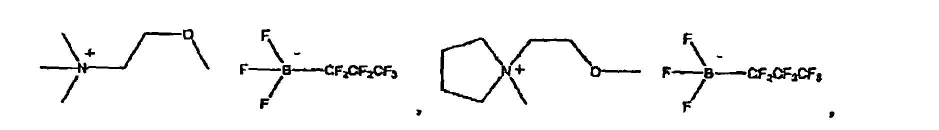

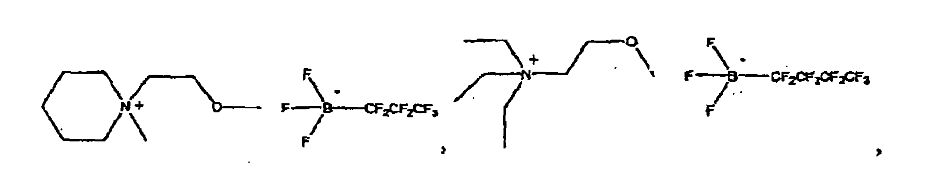

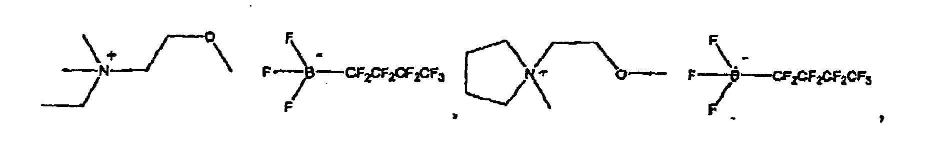

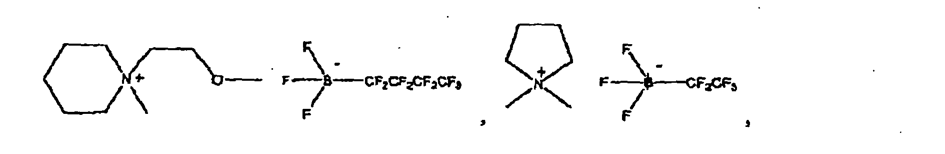

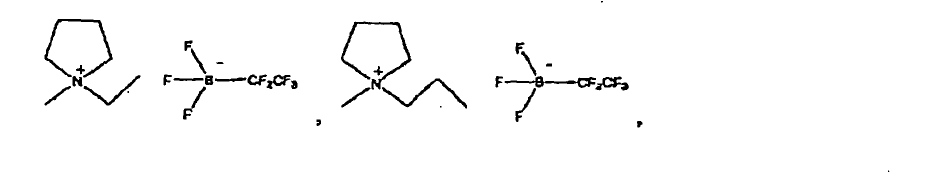

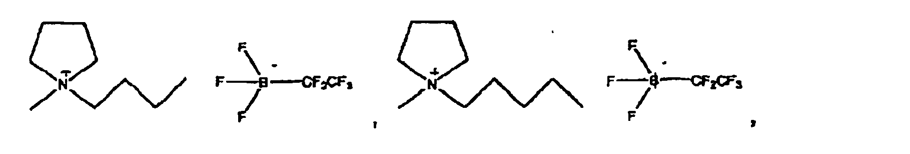

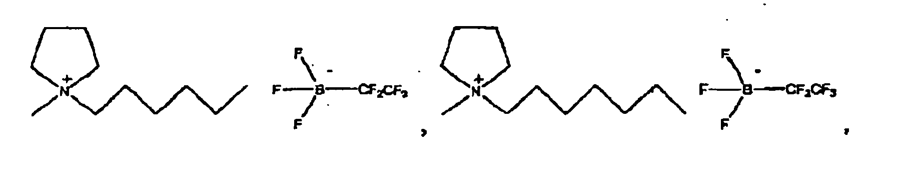

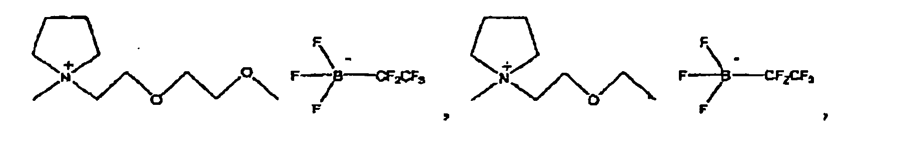

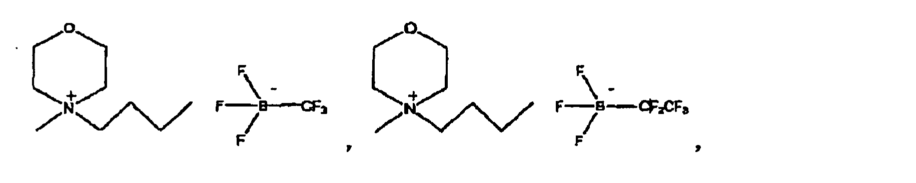

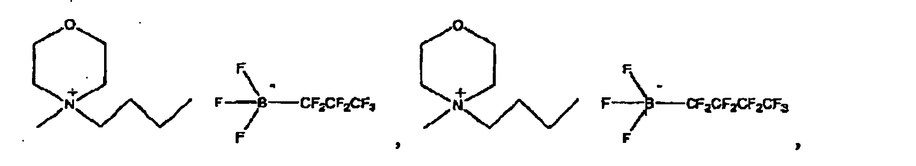

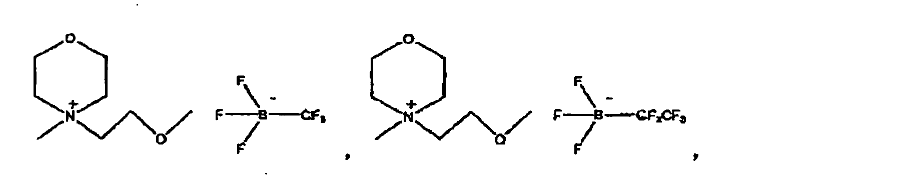

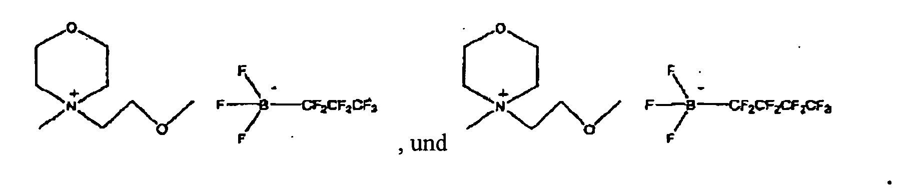

2. Elektrischer doppelschichtiger Kondensator umfassend die ionische Flüssigkeit nach Anspruch 1.

3. Lithiumbatterie umfassend die ionische Flüssigkeit nach Anspruch 1.

4. Verfahren zum Herstellen einer ionischen Flüssigkeit nach Anspruch 1, umfassend das Mischen einer Verbindung enthaltend als anionische Komponente mindestens ein Anion dargestellt durch [BF₃(CₙF₂ₙ+))]⁻, wobei n 1, 2, 3 oder 4 darstellt, mit einer Verbindung, die als kationische Komponente mindestens ein organisches Ammoniumion enthält, die gleich oder verschieden sind, wobei jedes aus der Gruppe ausgewählt ist bestehend aus 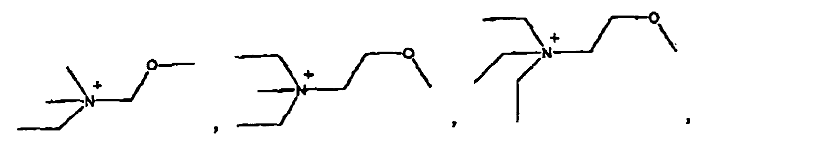 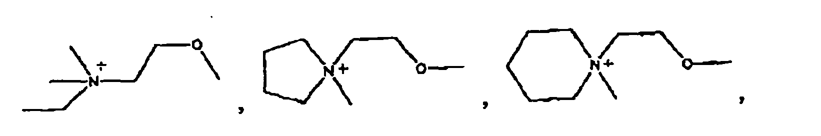 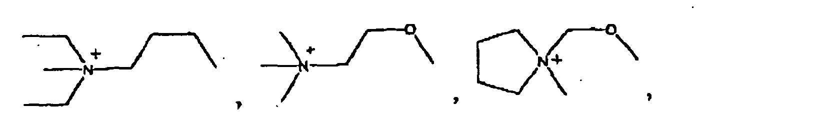 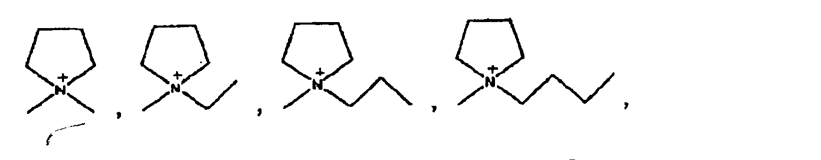 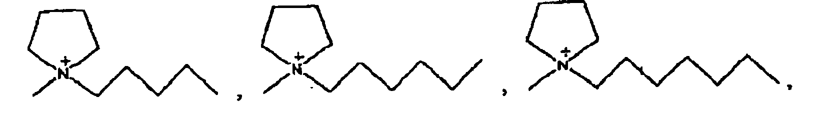 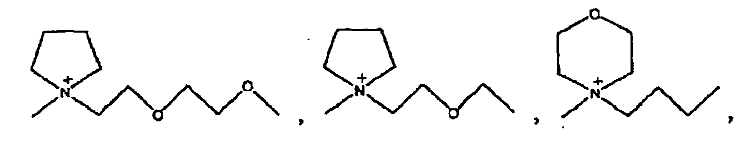 und

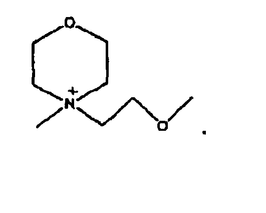

## Revendications

1. Liquide ionique comprenant au moins un élément choisi dans le groupe constitué de:

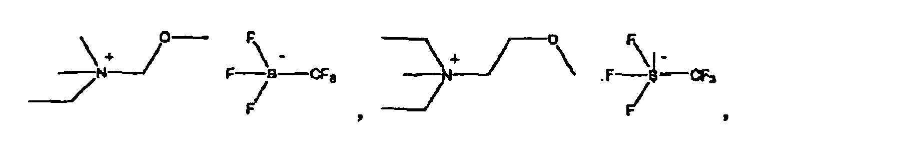

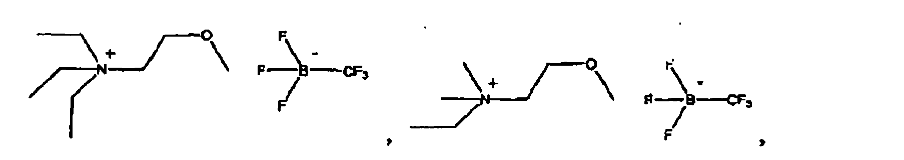

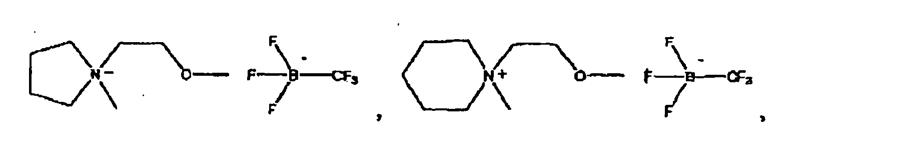

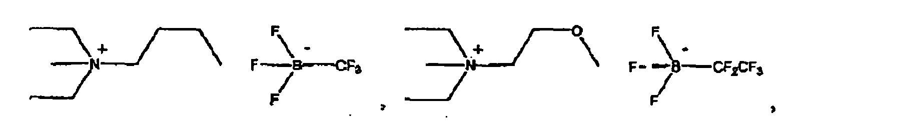

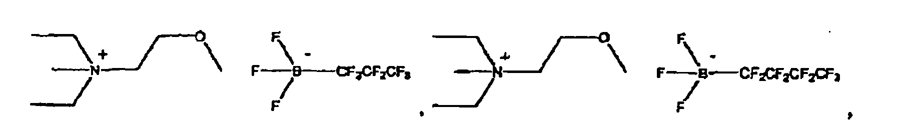

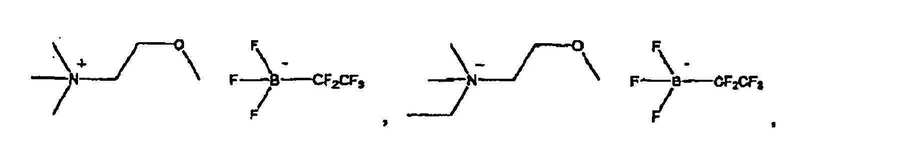

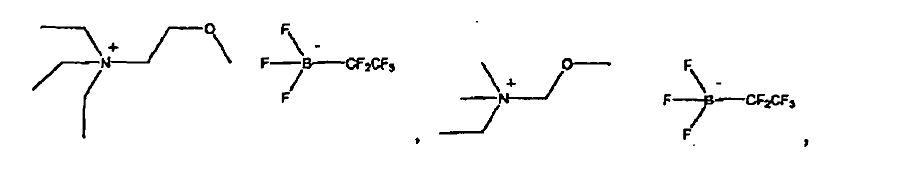

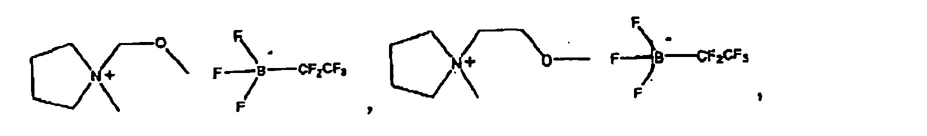

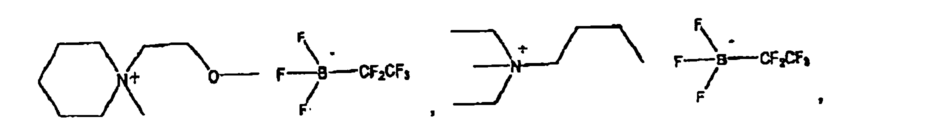

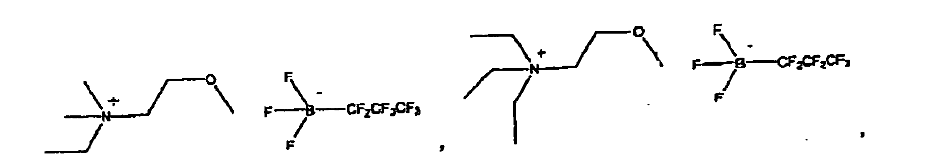

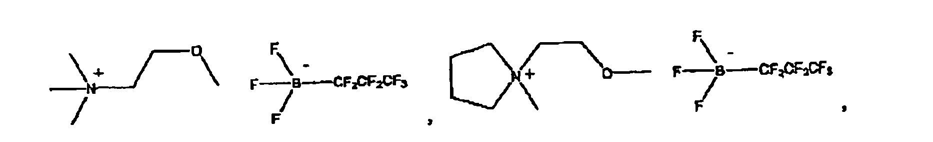

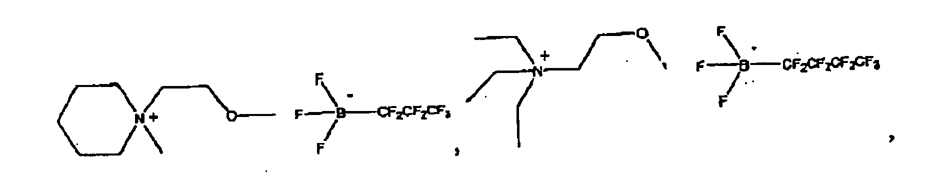

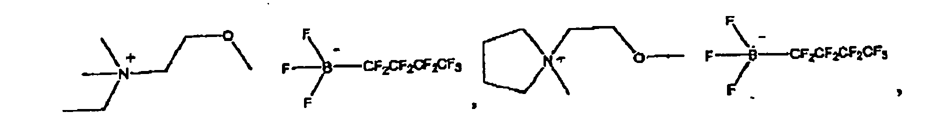

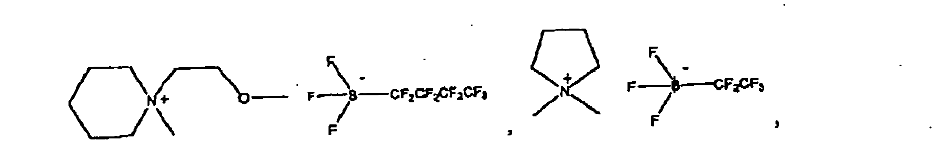

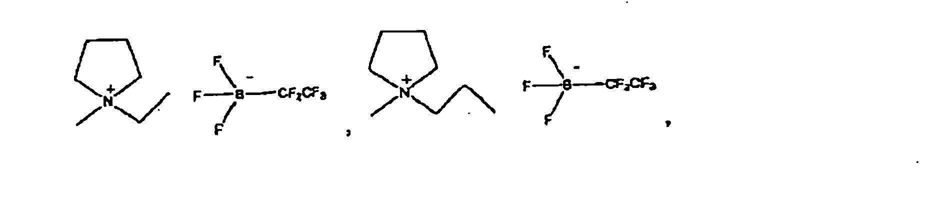

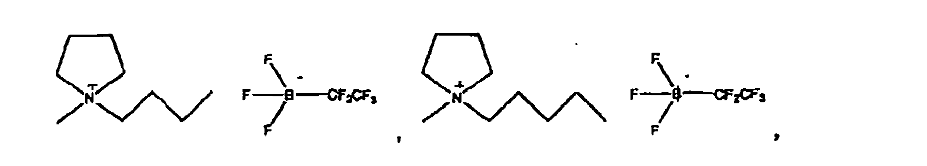

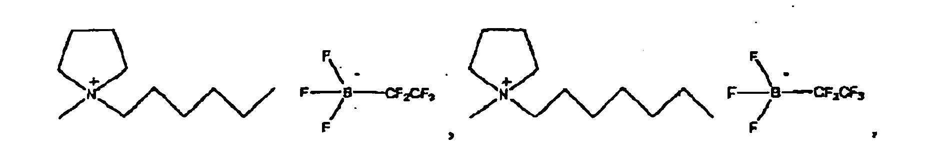

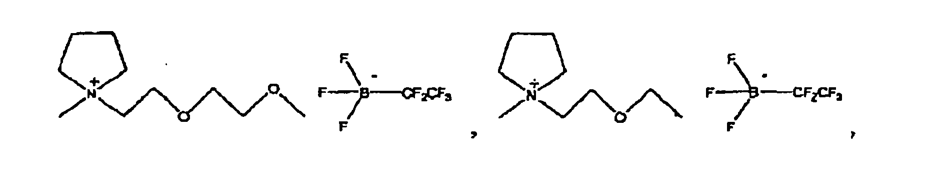

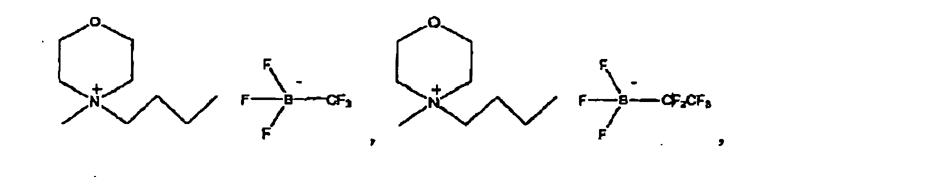

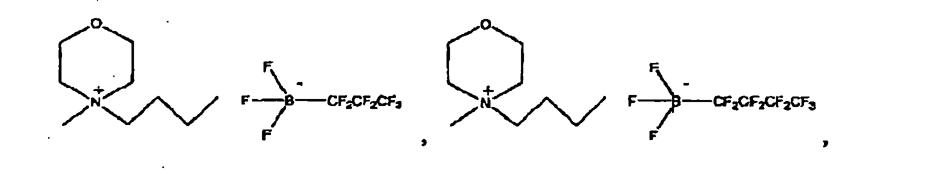

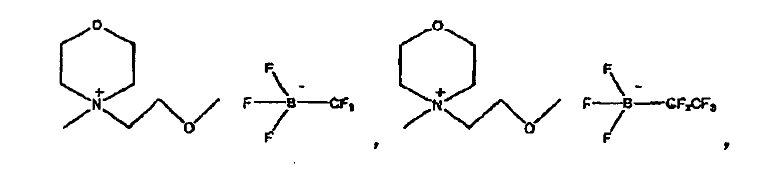

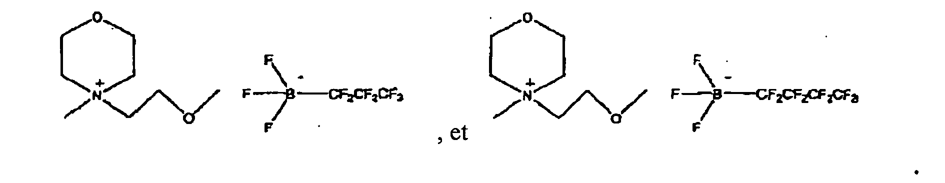

2. Condensateur double-couche électrique comprenant le liquide ionique selon la revendication 1.

3. Pile au lithium comprenant le liquide ionique selon la revendication 1.

4. Procédé pour la production d'un liquide ionique selon la revendication 1 comprenant le mélange d'un composé contenant en tant que composant anionique au moins un anion représenté par [BF₃(CₙF₂ₙ₊₁)]⁻, où n représente 1, 2, 3 ou 4, avec un composé contenant en tant que composant cationique au moins un ion ammonium organique qui sont identiques ou différents, chacun étant choisi dans le groupe constitué de: 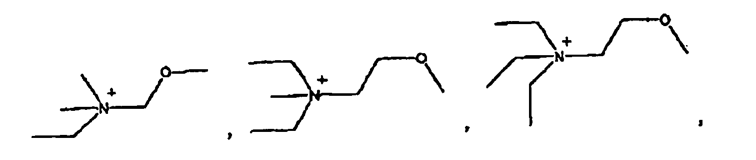 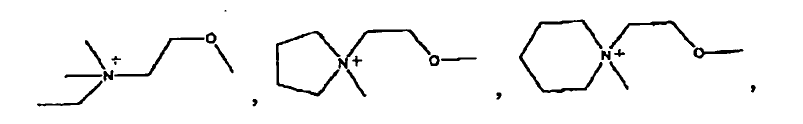 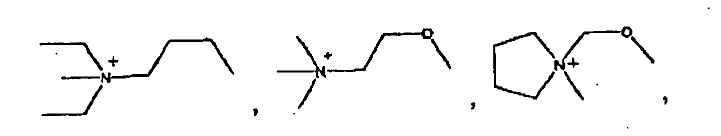 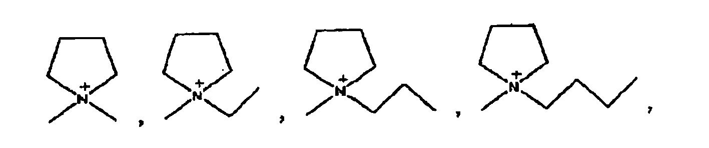 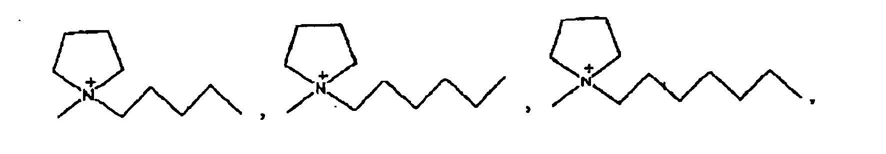 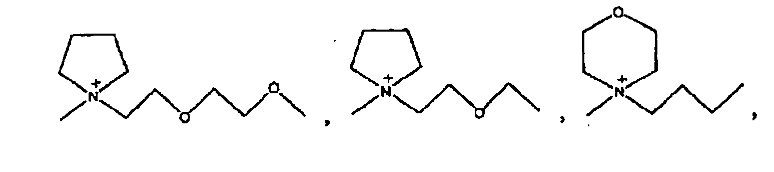 et
